# EUROPEAN PATENT APPLICATION

(11) **EP 4 043 030 A1**
(43) Date of publication of application: **17.08.2022**
(21) Application number: 20873520.9
(22) Date of filing: 27.03.2020
(51) Int. Cl.: A61K 39/12, A61P 31/12, A61P 37/04, C12N 5/10, C12N 7/00, C12N 7/01, C12N 15/37, C12N 15/54, C12Q 1/04, C12Q 1/6897

(54) **PRODUCTION METHOD AND DETECTION METHOD OF AFRICAN SWINE FEVER VIRUS**

(30) Priority: 11.10.2019 JP 2019187750
(71) Applicant: NATIONAL AGRICULTURE AND FOOD RESEARCH ORGANIZATION, Tsukuba-shi, Ibaraki 305-8517 (JP)
(72) Inventor: MASUJIN, Kentaro, Kodaira-shi, Tokyo 187-0022 (JP); TAKENOUCHI, Takato, Tsukuba-shi, Ibaraki 305-8634 (JP); KOKUHO, Takehiro, Kodaira-shi, Tokyo 187-0022 (JP); UENISHI, Hirohide, Tsukuba-shi, Ibaraki 305-8634 (JP); KITANI, Hiroshi, Tsukuba-shi, Ibaraki 305-8634 (JP); KAMEYAMA, Kenichiro, Kodaira-shi, Tokyo 187-0022 (JP)
(74) Representative: J A Kemp LLP
(86) International application number: PCT/JP2020/013953
(87) International publication number: WO 2021/070407

(57) **Abstract**

According to the present invention, immortalized cells derived from porcine kidney macrophages are found to have high infection susceptibility to various African swine fever virus strains. As a result, using the cells, it is possible to proliferate the virus and furthermore produce and detect the virus.

## Description

### [Technical Field]

The present invention relates to a method for producing African swine fever virus (ASFV), and more particularly to a method for producing ASFV using immortalized porcine kidney macrophages. The present invention also relates to a method for producing a vaccine containing ASFV using immortalized porcine kidney macrophages. Further, the present invention relates to immortalized porcine kidney macrophages containing DNA in which a reporter gene is functionally bound to a position downstream of a promoter region of an ASFV-derived gene, and a method for detecting ASFV using the immortalized porcine kidney macrophages.

### [Background Art]

African swine fever (ASF) is a highly lethal swine infectious disease caused by ASF virus (ASFV) infection. The outbreak of ASF has not yet been observed in Japan. However, it is feared that, if it occurs, the impact on the livestock industry will be enormous because there is no effective detection method and there is no effective vaccine or treatment method.

What is necessary for developing methods for detecting and preventing ASFV is an environment infected with the virus to proliferate the virus. However, it is not easy to conduct an ASFV infection test on individual swines, which are large farm animals. For this reason, an in vitro virus proliferation system (hereinafter referred to as the in vitro system) using cultured cells is extremely useful also for ASFV infection.

Since ASFV targets macrophages in swine bodies, the in vitro system for ASFV uses primary cultured macrophages. However, the primary cultured macrophages have not only drawbacks that may affect the reliability of experiments, such as variations in properties among collected lots and the high possibility of contamination with foreign viruses, but also problems in terms of animal welfare and cost such as the necessity of the sacrificial deaths of many swines to obtain a sufficient number of cells for conducting an infection test because the primary cultured macrophages do not proliferate after being collected from the swines.

In order to avoid these problems, the use of passageable cell lines has been considered. As a cell line having infection susceptibility to ASFV, a monkey-derived epithelial cell line (such as Vero cells) is mainly used. However, it is known that when the monkey-derived epithelial cell line, which is not the original host, is used, most of the ASFV field strains cannot establish infection on this cell line, and even if ASFV establishes infection, the ASFV exhibits poor proliferation efficiency and cannot maintain its original properties due to a mutation of virus genome during the proliferation or the like.

For this reason, there is a demand for the production of immortalized porcine-derived macrophage cell lines which are highly safe and proliferative with homogeneous properties. As porcine macrophage cell lines, cell lines prepared by introducing an immortalizing gene in the form of a plasmid vector into porcine alveolar macrophages using a transfection reagent have been reported (PLT 1 and NPL 1). However, these porcine macrophage cell lines have low infection susceptibility to ASFV and poor proliferation efficiency. Thus, any satisfactory cell line has not been found yet.

### [Citation List]

### [Patent Literature]

[PTL 1] International Application Japanese-Phase Publication No. 2017-500029

### [Non Patent Literature]

[NPL 1] Weingartl HM. et al., J Virol Methods, July 2002, Vol. 104(2), pp. 203-216
[NPL 2] Takenouchi T. et al., Front Vet Sci., 21 August 2017; 4:132.

### [Summary of Invention]

### [Technical Problem]

The present invention was made in view of the above problems in the related art, and has an object to find immortalized porcine-derived macrophage cells having high infection susceptibility to African swine fever virus, and to provide a method for proliferating African swine fever virus using the cells.

### [Solution to Problem]

The present inventors earnestly studied to achieve the above object and conducted an infection test on various cells using various African swine fever virus (ASFV) strains. As a result, it was revealed that immortalized porcine alveolar macrophage-derived cells (3D4/21, see NLP 1) exhibited susceptibility to ASFV strains acclimatized to cultured cells but did not exhibit susceptibility at all to field strains used in this infection test, whereas the immortalized porcine kidney macrophages (IPKM, see NPL 2), which had been previously developed by the present inventors, had susceptibility to all the ASFV strains used in the test and allowed proliferation of these virus strains. In addition, the susceptibility was, surprisingly, 10 to 100 times higher than that of the primary cultured porcine alveolar macrophages. Thus, the present inventors found that the immortalized porcine kidney macrophage-derived cells have high infection susceptibility to ASFV, and completed the present invention.

In sum, the present invention relates to a method for producing ASFV or a vaccine using immortalized porcine kidney macrophages. In addition, the present invention relates to immortalized porcine kidney macrophages containing DNA in which a reporter gene is functionally bound to a position downstream of a promoter region of an ASFV-derived gene, and a method for detecting ASFV using the immortalized porcine kidney macrophages, and specific description thereof is given below.
<1> A method for producing African swine fever virus comprising the step of bringing an immortalized porcine kidney macrophage into contact with African swine fever virus to proliferate the virus in the immortalized porcine kidney macrophage.
<2> A method for producing a vaccine containing African swine fever virus, comprising the steps of:
   bringing an immortalized porcine kidney macrophage into contact with African swine fever virus to proliferate the virus in the immortalized porcine kidney macrophage;
   isolating the African swine fever virus proliferated; and
   mixing the isolated African swine fever virus with a pharmacologically acceptable carrier or medium.
<3> The production method according to <1> or <2>, in which the immortalized porcine kidney macrophage is an immortalized macrophage obtained by introducing lentivirus encoding SV40 large T antigen and porcine-derived telomerase reverse transcriptase into a porcine-derived kidney macrophage.
<4> An immortalized porcine kidney macrophage infected with African swine fever virus.
<5> An immortalized porcine kidney macrophage which is obtained by introducing lentivirus encoding SV40 large T antigen and porcine-derived telomerase reverse transcriptase into a porcine-derived kidney macrophage, and which is infected with African swine fever virus.
<6> An immortalized porcine kidney macrophage comprising DNA in which a reporter gene is functionally bound to a position downstream of a promoter region of an African swine fever virus-derived gene.
<7> An immortalized porcine kidney macrophage which is obtained by introducing lentivirus encoding SV40 large T antigen and porcine-derived telomerase reverse transcriptase into a porcine-derived kidney macrophage, and which comprises DNA in which a reporter gene is functionally bound to a position downstream of a promoter region of an African swine fever virus-derived gene.
<8> A method for detecting African swine fever virus comprising the following steps of:
   culturing the immortalized porcine kidney macrophage according to <6> or <7> in the presence of a test sample;
   detecting expression of the reporter gene in the immortalized porcine kidney macrophage; and
   determining that the test sample contains African swine fever virus when the expression of the reporter gene is detected.

### [Advantageous Effects of Invention]

According to the present invention, it is possible to proliferate various African swine fever viruses (ASFVs) using immortalized porcine kidney macrophages having high infection susceptibility to ASFVs, and to therefore produce and develop vaccines against the viruses. In addition, detection (examination and diagnosis) of ASFV is also possible owing to the high infection susceptibility.

### [Brief Description of Drawings]

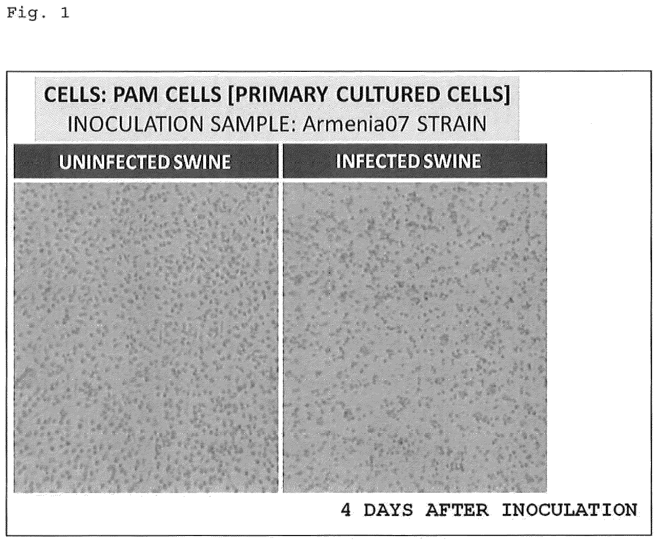

[Fig. 1] Fig. 1 is a micrograph showing a result of detection of cytopathic effect (CPE) in observation of primary cultured porcine alveolar macrophage cells (PAM cells) 4 days after inoculation with the African swine fever virus (ASFV) strain, the Armenia07 strain. In Fig. 1, the left panel "Uninfected Swine" shows a result of a negative control not inoculated with the ASFV strain.

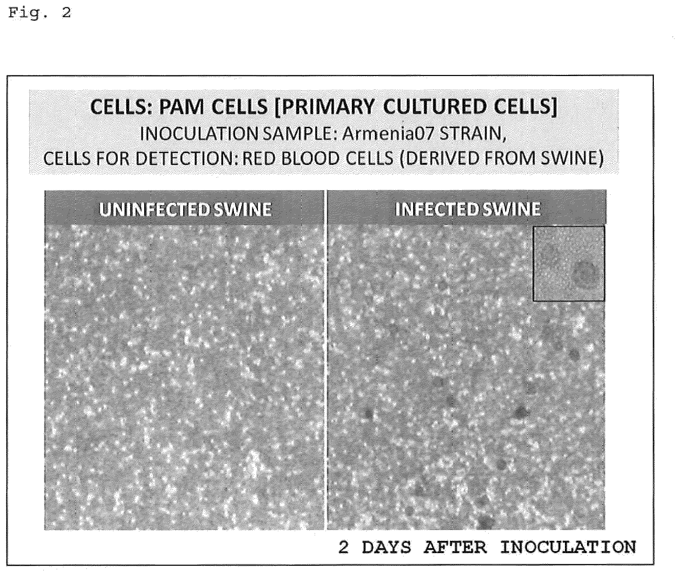

[Fig. 2] Fig. 2 is a micrograph showing a result of detection of a hemadsorption (HAD) reaction in observation of PAM cells 2 days after inoculation with the Armenia07 strain in the presence of red blood cells. In Fig. 2, the left panel "Uninfected Swine" shows a result of a negative control not inoculated with the ASFV strain.

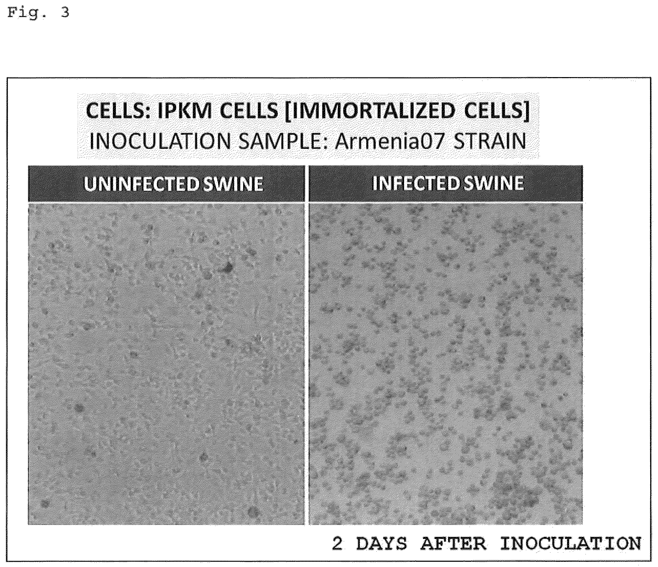

[Fig. 3] Fig. 3 is a micrograph showing a result of detection of CPE) in observation of immortalized porcine kidney macrophage cells (IPKM cells) 2 days after inoculation with the Armenia07 strain. In Fig. 3, the left panel "Uninfected Swine" shows a result of a negative control not inoculated with the ASFV strain.

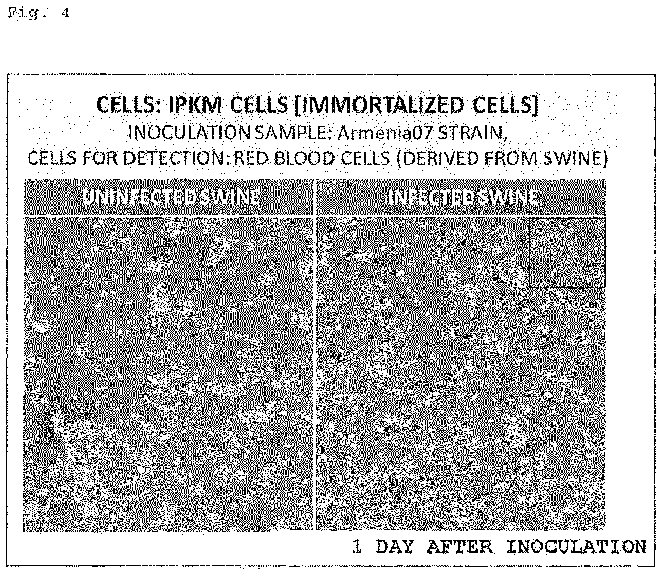

[Fig. 4] Fig. 4 is a micrograph showing a result of detection of a HAD reaction in observation of IPKM cells 1 day after inoculation with the Armenia07 strain in the presence of red blood cells. In Fig. 4, the left panel "Uninfected Swine" shows a result of a negative control not inoculated with the ASFV strain.

### [Description of Embodiments]

As shown in Example below, immortalized porcine kidney macrophages exhibit high infection susceptibility to African swine fever virus and enable proliferation of the virus.

Accordingly, the present invention provides a method for producing African swine fever virus including the step of bringing immortalized porcine kidney macrophages into contact with African swine fever virus to proliferate the virus in the immortalized porcine kidney macrophages. In addition, the present invention also provides immortalized porcine kidney macrophages infected with African swine fever virus.

### (Immortalized Porcine kidney Macrophages)

In the present invention, "immortalized porcine kidney macrophages" which serve as a place for infection and proliferation of African swine fever virus mean immortalized cells of macrophages present in the kidney of a swine (an animal in Mammalia, Cetartiodactyla, Suidae) .

The "porcine kidney macrophages" to be immortalized can be prepared by those skilled in the art according to a known method. For example, as described in Takenouchi T. et al., Results Immunol., August 2014, Issue 1, Vol. 4, pp. 62 to 67, the porcine kidney macrophages may be prepared by: removing the fibrous renal capsule from the kidneys dissected out from swine neonates; isolating the renal cortex; mincing the isolated renal cortex; and then isolating macrophage-like cells which are produced in the process of culturing the minced renal cortex, are loosely attached to a monolayer cell sheet, and exhibit a proliferative activity.

The method for immortalizing porcine kidney macrophages is not particularly limited, and may be implemented by introducing at least one kind of immortalizing genes. Examples of the immortalizing genes include SV40 large T antigen (SV40 T antigen), telomerase reverse transcriptase (TERT), Myc, and Ras, and it is preferable to introduce SV40 T antigen and TERT. From the viewpoint that the immortalization efficiency of porcine macrophages can be enhanced, it is more preferable to introduce the SV40 T antigen and porcine-derived TERT.

The introduction of the immortalizing gene may be carried out by using a vector encoding the gene. The vector may be linear or circular, and examples thereof include a viral vector, a plasmid vector, an episomal vector, an artificial chromosome vector, and a transposon vector.

Examples of the viral vector include retrovirus vector such as lentivirus, Sendai virus vector, adenovirus vector, adeno-associated virus vector, herpesvirus vector, vaccinia virus vector, poxvirus vector, poliovirus vector, sindbis virus vector, rhabdovirus vector, paramyxovirus vector, and orthomyxovirus vector. Examples of the plasmid vector include plasmid vectors for animal cell expression such as pcDNA3.1, pA1-11, pXT1, pRc/CMV, pRc/RSV, and pcDNAI/Neo. Among these vectors, retrovirus vector is preferable and lentivirus is more preferable from the viewpoint that the efficiency of gene introduction into porcine macrophages can be enhanced.

The vector according to the present invention may include substances other than the immortalizing gene, such as: expression control sequences such as promoters, enhancers, poly A addition signals, and terminators; nucleotide sequences encoding proteins that control replication by binding to the origin of replication, a 5' untranslated region including a 5' cap structure, the Shine-Dalgarno sequence, the Kozak sequence, and the like; a 3' untranslated region containing a polyadenylation signal, an AU-rich element, a GU-rich element, and the like; nucleotides encoding other proteins.

When being operably arranged downstream of the promoter, the immortalizing gene can efficiently transcribe polynucleotides. Examples of such "promoter" include EF1α promotor, CMV promotor, SRα promotor, SV40 early promotor, LTR promotor, RSV promotor, HSV-TK promotor, MSCV promotor, hTERT promotor, β-actin promoter, CAG promoter, metallothionein promoter, heat shock promoter, and the like.

Examples of the "nucleotides encoding other proteins" include marker genes such as a reporter gene and a drug resistant gene.

In the case where two or more kinds of immortalizing genes are introduced, these genes may be incorporated in a single vector or respectively different vectors. From the viewpoint that the expression efficiency can be enhanced, it is desirable that these genes be incorporated in different vectors. When being incorporated in a single vector, multiple kinds of immortalizing genes can be expressed polycistronically by inserting, for example, an IRES or 2A peptide sequence or the like into the vector.

As the method for introducing the vector into cells, there are lipofection method, microinjection method, calcium phosphate method, DEAE-dextran method, electroporation method, particle gun method, and so on. In the case where the vector of the present invention is a retrovirus vector, retroviral particles may be prepared by selecting and using appropriate packaging cells based on LTR sequences and a packaging signal sequence included in the vector. Examples of the packaging cells include PG13, PA317, GP+E-86, GP+envAm-12, and Psi-Crip. In addition, 293 cells and 293T cells, which have high transfection efficiency, may be used as the packaging cells. Moreover, the viral particles thus prepared may be introduced into cells according to a method such as Polybrene method, Protamine method, and RetroNectin method.

The immortalized porcine kidney macrophages established by introducing the immortalizing gene as described above exhibit proliferative properties for at least 1 month or longer, preferably 3 months or longer, and more preferably 5 months or longer. The doubling time for the immortalized porcine kidney macrophages is at least 6 days, preferably 4 days, and more preferably 2 days. Further, the immortalized porcine kidney macrophages preferably maintain macrophage properties. For example, among macrophage-specific genes CD172a, CD16, Iba-1, MSR-A, MHC-II, and CD163, at least one gene is expressed, preferably two genes are expressed, more preferably four genes are expressed, and particularly preferably all the genes are expressed. Moreover, the immortalized porcine kidney macrophages according to the present invention maintain, as the macrophage properties, at least one, preferably two, and more preferably all of functions of phagocytosis, production of inflammatory cytokines by LPS stimulation, and IL-1β maturation associated with inflammasome activity. The present invention particularly preferably uses immortalized macrophages that can have all the aforementioned properties, are produced according to the method described in Takenouchi T. et al., Front Vet Sci., 21 August 2017; 4:132 (NPL 2), and are obtained by introducing lentivirus encoding SV40 large T antigen and porcine-derived telomerase reverse transcriptase into porcine-derived kidney macrophages. It should be noted that the immortalized porcine kidney macrophages obtained according to the method described in NPL 2 develop densely and proliferate (in a sheet form) unlike the primary cultured porcine macrophages. Therefore, a morphological change in the macrophages can be easily detected, so that the macrophages are also useful in African swine fever virus infection tests (such as CPE test and HAD test) using degeneration of infected cells as an index, which will be described later.

### (African Swine Fever Virus)

African swine fever virus (Asfarviridae Asfivirus, ASFV) is a virus of the genus Asfivirus in the family Asfarviridae having double-stranded DNA in its genome. In the present invention, African swine fever virus with which the aforementioned immortalized porcine kidney macrophages are to be infected is not particularly limited, and may be of any of the 24 reported genotypes (such as type II, type I, and type X). The African swine fever virus may have any level of pathogenicity (for example, it may be high- or low- virulence virus or be extremely acute, acute, subacute, chronic or subclinical fever virus) and be of a field type or an acclimatized type without particular limitation, and can proliferate by infecting the aforementioned immortalized porcine kidney macrophages.

### (Method for Producing ASFV)

The method for producing ASFV (proliferation method or amplification method) in the present invention is a method including the step of bringing immortalized porcine kidney macrophages into contact with ASFV to proliferate the virus in the immortalized porcine kidney macrophages.

The ASFV to be brought into contact with the immortalized porcine kidney macrophages is as described above, and may be the isolated virus itself or a sample that may contain the virus. As such "sample", there are porcine-derived tissues or cells, a culture, washing solution, or extract thereof, a washing solution of a swine breeding environment (such as a breeding facility), or a culture thereof.

The "contact" may be usually conducted by adding the ASFV to a medium where the immortalized porcine kidney macrophages are cultured. Such "medium" is not particularly limited as long as it is a medium that can maintain the immortalized porcine kidney macrophages, and may be prepared based on a known basal medium by adding a well-known and common medium additive thereto. As the "basal medium", there are DMEM medium, DMEM medium (high glucose), DMEM medium (low glucose), RPMI 160 medium, RPMI 1640 medium, ham F12 medium, KSOM medium, Eagle MEM medium, Glasgow MEM medium, αMEM medium, ham medium, Fishers medium, BME medium, BGJb medium, CMRL 1066 medium, MEM Zinc option improvement medium, IMDM medium, medium 199 medium, and a mixture of any two or more of these media. Examples of the "medium additive" include, but are not limited to, antibiotics (such as penicillin, streptomycin, and gentamicin), antifungal agents, functional proteins (such as insulin, transferase, and lactoferrin), reducing agents (such as 2-mercaptoethanol, monothioglycerol, catalase, superoxide dismutase, and N-acetylcysteine), lipids other than fatty acids (such as cholesterol), amino acids (such as alanine, L-glutamine, and non-essential amino acids), peptides (such as glutathione and reduced glutathione), nucleotides and the like (such as nucleoside, cytidine, adenosine 5'-monophosphate, hypoxanthine, and thymidine), metal salts (such as iron nitrate (III), iron sulfate (II), copper sulfate, and zinc sulfate), inorganic salts (such as sodium, potassium, calcium, magnesium, phosphorus, and chlorine), carbon sources (such as glucose, galactose, fructose, and sucrose), vitamins, inorganic compounds (such as selenous acid), organic compounds (such as paraaminobenzoic acid, ethanolamine, corticosterone, progesterone, lipoic acid, putrescine, pyruvate, lactic acid, and triiodothyronine), buffer compounds (such as HEPES and sodium bicarbonate), and pH indicators (such as phenol red).

The "proliferation" of ASFV can be achieved by culturing immortalized porcine kidney macrophages having been brought into contact with and infected with the virus. A culture temperature is not particularly limited but is usually 30 to 42°C and preferably 37°C. A concentration of carbon dioxide in a gas in contact with the medium is not particularly limited but is usually 1 to 10% by volume and preferably 2 to 5% by volume. A culture period after contact with ASFV is not particularly limited but is usually 1 to 10 days, preferably 2 to 7 days, and more preferably 3 to 5 days.

Whether ASFV proliferates or not may be determined by those skilled in the art according to a known method. Examples of such a method include a CPE test using the cytopathic effect (CPE) as an index and a HAD test using a hemadsorption (HAD) reaction specifically observed in ASFV-infected cells as an index as will be described below in Example. In addition, it is also possible to use a method for detecting a gene derived from ASFV or its expression. Here, the expression of the gene may be at a transcription level (mRNA level) or a translation level (protein level). Examples of the method for detecting the gene (genomic DNA) or mRNA include PCR (RT-PCR, real-time PCR, and quantitative PCR), DNA microarray analysis, northern blotting or southern blotting, in situ hybridization, dot blotting, RNase protection assay method, and a mass spectrometry method. Moreover, the gene or mRNA level can be quantitatively detected by counting the number of reads in the so-called next-generation sequencing method. Examples of the method for detecting a protein include detection methods using antibodies (immunological methods) such as an ELISA method, antibody array, immunoblotting, imaging cytometry, flow cytometry, radioimmunoassay, immunoprecipitation method, and immunohistochemical staining method, and mass analysis methods.

### (Method for Producing Vaccine)

The method for producing a vaccine containing ASFV of the present invention is a method including the steps of:
bringing immortalized porcine kidney macrophages into contact with African swine fever virus to proliferate the virus in the immortalized porcine kidney macrophages;
isolating the African swine fever virus proliferated; and
mixing the isolated African swine fever virus with a pharmacologically acceptable carrier or medium.

The step to proliferate the virus in the immortalized porcine kidney macrophages is as described above. The "isolating" of the ASFV proliferated means separation from the culture medium, purification, and/or condensation of the immortalized porcine kidney macrophages and/or the cells. Examples of the method for isolating the virus include filtration of the culture medium, cell disruption (such as ultrasonic treatment, hypotonic solution treatment, and freeze-thaw), centrifugation (such as ultracentrifugation and density gradient centrifugation), and condensation (such as ammonium sulfate, resin column, and polyethylene glycol salting out).

The ASFV thus isolated may be used directly as a vaccine (so-called live vaccine), be used in an attenuated live form (so-called live attenuated virus), or be used as a vaccine in an inactivated form. Further, part of the isolated ASFV (such as protein, polypeptide, sugar, glycoprotein, lipid, or nucleic acid) may be used as a vaccine as long as it has immunogenicity.

The live attenuated virus means a virus having a virulence level reduced as compared with viruses isolated from the field. The attenuated virus can be obtained by a known method, for example, by subjecting ASFV to proliferation in the presence of mutagens, acclimatization to cultured cells by continuous (long-term) passage in vitro, or proliferation under a condition deviating from the natural growth environment (for example, under a high temperature condition). In addition, the live attenuated virus may be obtained by deleting or recombining a specific gene of the virus using genome editing, gene modification technique, or the like.

The inactivation of the virus may be conducted by those skilled in the art using a known method. As such inactivation method, there are formaldehyde treatment, UV irradiation, X-ray irradiation, electron beam irradiation, gamma irradiation, alkylation treatment, ethylene-imine treatment, thimerosal treatment, β-propiolactone treatment, and glutaraldehyde treatment.

Examples of the "pharmacologically acceptable carrier" to be mixed with the isolated ASFV include stabilizers, excipients, preservatives, surfactants, chelating agents, and binders. Examples of the "pharmacologically acceptable medium" include water, saline, phosphate buffer solution, and Tris-HCl buffer solution. As these carriers and media, publicly-known ones for use in this technical field may be selected as appropriate or in combination depending on the dosage form and usage method of the vaccine. The form of the vaccine is not particularly limited, and may be, for example, a form of suspension or a form of freeze dry.

From the viewpoint that the effect of the vaccine can be enhanced, an adjuvant may be added further. Examples of the adjuvant include inorganic substances such as aluminum gel adjuvant, microorganisms or substances derived from microorganisms (such as BCG, muramyl dipeptide, pertussis, pertussis toxin, and cholera toxin), surfactant action substances (such as saponin and deoxycholic acid), emulsions of oily substances (such as mineral oil, vegetable oil, and animal oil), alum, and so on.

### (Method for Detecting ASFV)

As shown in Example below, the immortalized porcine kidney macrophages have high infection susceptibility to ASFV. For this reason, when a reporter system that is activated depending on ASFV infection and proliferation, in other words, DNA in which a reporter gene is functionally bound to a position downstream of a promoter region of an ASFV-derived gene is incorporated into the immortalized porcine kidney macrophages, these macrophages form a system useful for ASFV detection.

Therefore, the present invention provides immortalized porcine kidney macrophages including DNA in which a reporter gene is functionally bound to a position downstream of a promoter region of an ASFV-derived gene. The present invention further provides a method for detecting ASFV, including the steps of: culturing the immortalized porcine kidney macrophages including the DNA in the presence of a test sample; detecting expression of the reporter gene in the immortalized porcine kidney macrophages; and determining that the test sample contains African swine fever virus when the expression of the reporter gene is detected.

The "immortalized porcine kidney macrophages" into which the DNA is introduced are as described above. Then, the DNA may be in the form of any of the vectors described above in the "immortalizing genes". Moreover, the introduction of the DNA into the immortalized porcine kidney macrophages may be also conducted by those skilled in the art using any of the methods described in the above explanation about the "immortalizing genes".

The "promoter region of the ASFV-derived gene" in the DNA is not particularly limited as long as it is a region derived from ASFV and being capable of activating the expression of the gene downstream thereof in response to infection with and proliferation of the virus, and may be any gene among an immediate-early gene, an early gene, a late gene, and a very-late gene. The ASFV-derived gene for use may be selected by those skilled in the art in reference to appropriate known information (for example, the list of "Functions of ASFV-encoded proteins" presented in Table 1 on pp. 155 to 157 in Ninth Report of the International Committee on Taxonomy of Viruses (ICTV), 2012), and a preferred one for use is a promoter region of p72, U104L, CD2v, DNA polymerase, or p30 gene (see Portugal RS. et al., Virology, August 2017, Vol. 508, pp. 70 to 80).

The "reporter gene" functionally (operably) bound to the position downstream of the promoter region is not particularly limited and a known gene may be used as appropriate. Examples thereof include fluorescent protein genes, luminescent enzyme genes, and chromogenic enzyme genes. Specific ones as the fluorescent protein genes include GFP (green fluorescent protein) gene, YFP (yellow fluorescent protein) gene, RFP (red fluorescent protein) gene, and so on. Specific ones of the luminescent protein/enzyme genes include aequorin gene, luciferase gene, and so on. Specific ones of the chromogenic enzyme genes include chloramphenicol acetyltransferase (CAT) gene, β-glucuronidase (GUS) gene, β-galactosidase gene, alkaline phosphatase gene, SEAP gene, and so on.

By using, as an index, any of the fluorescence, luminescence, color development, and the like generated in response to the expression of these reporter genes, the detection method of the present invention is capable of detecting whether or not immortalized porcine kidney macrophages are infected with ASFV and furthermore detecting the presence of ASFV in a test sample.

The test sample is not particularly limited as long as it is a sample where ASFV can exist, and examples thereof include porcine-derived tissues or cells, a culture, washing solution, or extract thereof, a washing solution of a swine breeding environment (such as a breeding facility), or a culture thereof. The culture temperature in the detection method of the present invention is not particularly limited but is usually 30 to 42°C and preferably 37°C. A concentration of carbon dioxide in a gas in contact with the medium is not particularly limited but is usually 1 to 10% by volume and preferably 2 to 5% by volume. A culture period until the expression of the reporter gene is detected in the presence of a test sample is not particularly limited, but is usually 1 to 10 days, preferably 2 to 7 days, and more preferably 2 to 5 days.

### Example

Hereinafter, the present invention will be described in more details based on Example, but the present invention should not be limited to the following Example. In Example, the virus susceptibility of immortalized porcine kidney macrophage (IPKM) cells to ASFV was evaluated by using the following cells and African swine fever viruses (ASFVs).

### (Cells)

As IPKM cells of the cell line created by immortalizing porcine kidney macrophage cells, cells previously established by the present inventors (see NPL 2) were used. Porcine alveolar macrophage (PAM) cells were collected from porcine lungs according to the previous report (Carrascosa AL. et al., Curr Protoc Cell Biol, December 2011, Chapter 26, UNIT 26.14, pp. 1 to 26). Vero (CCL-81) cells and COS-1 (CRL-1650) cells of immortalized African green monkey kidney-derived cell lines and 3D4/21 (CRL-2843) cells of an immortalized porcine alveolar macrophage-derived cell line were introduced from the American Type Culture Collection (ATCC). As WSL cells of a wild boar lung-derived cell line, cells gifted from the Friedrich Loeffler Institute (FLI, Germany) were used.

Regarding cultures of these kinds of cells, a medium in which gentamicin (50 µg/mL) and penicillin-streptomycin (1:100) were added to RPMI 160 containing 10% fetal bovine serum (FBS) was used for the PAM cells. For the IPKM cells, used was a medium in which gentamicin (50 µg/mL), penicillin-streptomycin (1:100), insulin (10 µg/mL), monothioglycerol (25 µM), and Fungin (5 µg/mL) were added to 10% FBS-containing DMEM High glucose. For the Vero cells and the COS-1 cells, used were media in each of which gentamicin (50 pg/mL) and penicillin-streptomycin (1:100) were added to 5% FBS-containing DMEM. For the WSL cells and the 3D4/21 cells, used were media in each of which non-essential amino acids (1:100) and kanamycin (50 pg/mL) were added to 10% FBS-containing RPMI 1640.

### (ASFV)

Three ASFV field isolates: European/Chinese epidemic strain Armenia07 (genotype: type II), Kenya05/Tk-1 (genotype: type I) isolated from soft ticks, and ASFV standard strain Espana75 (genotype: type I) were introduced from the Complutense University of Madrid (Spain), the ASF Reference Laboratory of the International Epizootic Office (OIE). For information on the year and place of occurrence of each virus strain, see Fernandez-Pinero J. et al., Transbound Emerg Dis., February 2013, Vol. 60, Issue. 1, pp. 48 to 58. The Vero cell-acclimatized strain Lisbon60/V was introduced from Plum Island Animal Disease Center (PIADC, USA).

The Armenia07, Kenya05/Tk-1, and Espana75 strains were cultured to proliferate using the PAM cells, and the Lisbon60/V strains was cultured to proliferate using the Vero cells to prepare inoculation virus samples. The inoculation virus samples were dispensed and stored at -80°C.

### (Virus Susceptibility Test)

The virus susceptibility of cells was determined by a CPE test using the cytopathic effect (CPE) as an index or a HAD test using a hemadsorption (HAD) reaction specifically observed in ASFV-infected cells.

Specifically, the PAM cells (1×10⁵ cells), the Vero cells (1.5×10⁴ cells), the COS-1 cells (1.5×10⁴ cells), the WSL cells (2×10⁴ cells), the 3D4/21 cells (2×10⁴ cells), and the IPKM cells (3×10⁴ cells) per well were seeded in 96-well plates and pre-cultured in the above media for 1 to 2 days. After that, 25 µL of each of virus solutions diluted with the same medium at 10-fold serial dilution levels from 10-fold (10⁻¹) to 10 billion-fold (10⁻¹⁰) was inoculated into 8 wells to prepare a CPE detection plate. A HAD detection plate was prepared by further adding 20 µL of a suspension of 0.75% porcine erythrocyte suspended in phosphate buffered saline (PBS). The CPE detection plates and the HAD detection plates were subjected to culturing for 7 days in an environment at 37°C with 5% CO₂ and then were observed.

### (Results)

The above kinds of cells were compared using the CPE as the index in terms of the susceptibility to ASFV by use of the four different ASFV strains. The obtained results are shown in Table 1.

**[Table 1]**

| | **Armenia07 II type European/ Chinese epidemic strain** | **Kenya/Th-1 X Type Tick-derived strain** | **Espana75 I Type Standard strain** | **Lisbon60/V I type Cell-acclimatized strain** |
|---|---|---|---|---|
| **PAM (Alveolar macrophage cell)** | + | + | + | + |
| **Vero (African green monkey kidney)** | - | - | - | + |
| **COS-1 (African** green **monkey kidney)** | - | - | - | + |
| **WSL (Wild boar tung)** | - | - | - | + |
| **3D4/21 (Porcine alveolar macrophage cell)** | - | - | - | + |
| **IPKM (Porcine kidney macrophage cell)** | + | + | + | + |

As shown in Table 1, the CPE in the PAM cells and the IPKM cells was detected with all the inoculated virus strains (for example, see Figs. 1 and 3). On the other hand, the CPE in the Vero cells, the COS-1 cells, the WSL cells, and the 3D4/21 cells was not observed with the three field strains but was observed only with the cell-acclimatized strain Lisbon60/V.

In addition, as similar to the PAM cells, the IPKM cells were also revealed to exhibit the HAD properties with respect to the ASFV strains in addition to the CPE (see Figs. 2 and 4) .

Next, the dilution limit (virus detection limit) at which the virus could be separated was evaluated for both the PAM cells and the IPKM cells. The obtained results are shown in Tables 2 and 3.

**[Table 2]**

| | | Armenia07 | Kenya/Tk-1 | Espana75 | Lisbon60/V |
|---|---|---|---|---|---|
| PAM | Porcine alveolar macrophage (Primary cultured cell) | 10⁻⁴ | 10⁻³ | 10⁻³ | 10⁻³ |
| IPKM | Porcine kidney macrophage (immortalized cell) | 10⁻⁶ | 10⁻⁵ | 10⁻⁵ | 10⁻⁷ |

**[Table 3]**

| | | Armenia07 | Kenya/Tk-1 | Espana75 | Lisbon60/V |
|---|---|---|---|---|---|
| PAM | Porcine alveolar macrophage (Primary cultured cell) | 10⁻⁵ | 10⁻⁵ | 10⁻⁵ | 10⁻⁵ |
| IPKM | Porcine kidney macrophage (Immortalized cell) | 10⁻⁶ | 10⁻⁶ | 10⁻⁶ | 10⁻⁷ |

In the CPE test, as shown in Table 2, the detection limit concentration of the IPKM cells was 100 to 1000 times lower than that of the PAM cells. Similarly also in the HAD test, as shown in Table 3, the detection limit concentration of the IPKM cells was 10 to 100 times lower than that of the PAM cells. This revealed that the IPKM cells have higher susceptibility to ASFV than the PAM cells have.

### [Industrial Applicability]

As described above, according to the present invention, it is possible to proliferate various African swine fever viruses (ASFVs) using immortalized porcine kidney macrophages having high infection susceptibility to ASFVs, and to therefore produce and develop vaccines against the viruses. In addition, detection (examination and diagnosis) of ASFV is also possible owing to the high infection susceptibility.

## Claims

1. A method for producing African swine fever virus comprising the step of bringing an immortalized porcine kidney macrophage into contact with African swine fever virus to proliferate the virus in the immortalized porcine kidney macrophage.

2. A method for producing a vaccine containing African swine fever virus, comprising the steps of:
bringing an immortalized porcine kidney macrophage into contact with African swine fever virus to proliferate the virus in the immortalized porcine kidney macrophage;
isolating the African swine fever virus proliferated; and
mixing the isolated African swine fever virus with a pharmacologically acceptable carrier or medium.

3. The production method according to claim 1 or 2, wherein the immortalized porcine kidney macrophage is an immortalized macrophage obtained by introducing lentivirus encoding SV40 large T antigen and porcine-derived telomerase reverse transcriptase into a porcine-derived kidney macrophage.

4. An immortalized porcine kidney macrophage infected with African swine fever virus.

5. An immortalized porcine kidney macrophage which is obtained by introducing lentivirus encoding SV40 large T antigen and porcine-derived telomerase reverse transcriptase into a porcine-derived kidney macrophage, and which is infected with African swine fever virus.

6. An immortalized porcine kidney macrophage comprising DNA in which a reporter gene is functionally bound to a position downstream of a promoter region of an African swine fever virus-derived gene.

7. An immortalized porcine kidney macrophage which is obtained by introducing lentivirus encoding SV40 large T antigen and porcine-derived telomerase reverse transcriptase into a porcine-derived kidney macrophage, and which comprises DNA in which a reporter gene is functionally bound to a position downstream of a promoter region of an African swine fever virus-derived gene.

8. A method for detecting African swine fever virus comprising the following steps of:
culturing the immortalized porcine kidney macrophage according to claim 6 or 7 in the presence of a test sample;
detecting expression of the reporter gene in the immortalized porcine kidney macrophage; and
determining that the test sample contains African swine fever virus when the expression of the reporter gene is detected.
